Europäisches Patentamt

European Patent Office

Office européen des brevets

(19)

(11) **EP 1 234 554 A1**

(12) **EUROPEAN PATENT APPLICATION**

(43) Date of publication:
28.08.2002 Bulletin 2002/35

(51) Int Cl.$^7$: **A61F 2/06**

(21) Application number: **01104106.8**

(22) Date of filing: **21.02.2001**

(84) Designated Contracting States:
**AT BE CH CY DE DK ES FI FR GB GR IE IT LI LU
MC NL PT SE TR**
Designated Extension States:
**AL LT LV MK RO SI**

(71) Applicant: **EndoArt SA
1015 Lausanne (CH)**

(72) Inventor: **Stergiopoulos, Nikos
1025 Saint Sulpice (CH)**

(74) Representative: **Micheli, Michel-Pierre
MICHELI & CIE,
Rue de Genève 122,
Case Postale 61
1226 Genève-Thonex (CH)**

(54) **Vascular graft with internal deflector**

(57)    Graft for replacing or bypassing a damaged blood passage in a human or animal body, comprising an elongated tubular body (1) in which a flow deflector element (2) is maintained and stabilized in a substantially central position thanks to stabilizing rods (3). The flow deflector element (2) deviates the blood flow and thus increases the shear stress on the internal wall of the tubular element (1) so that the observed phenomena of graft's occlusion by thrombosis substantially decreases.

Fig.1

**Description**

The present invention relates to an implantable device in a human or animal body and more particularly to a synthetic vascular graft commonly used to replace or to bypass damaged or dysfunctional arterial or venous pathways.

Coronary artery bypass surgery requires the insertion of either natural or artificial substitute vessels to bypass one or more blocked arteries in the heart for example. Natural substitute vessels are the patient's saphenous veins or the internal mammary artery. However, these vessels may not be an option for patients who have undergone previous bypass surgery or who do not have vessels of appropriate quality. Clinical experiences show that small-bore grafts made out of conventional graft synthetic material, like polytetrafluoroethylene (ePTFE) occlude by thrombosis and thus are unable to maintain long-term patency.

There exists a strong need for the development of vascular grafts that can be used as substitute vessels for coronary bypass surgery or other peripheral indications. It is therefore an object of the invention to provide an artificial graft that can be used in bypass surgery without having the above mentioned drawbacks.

The foregoing and other objects of this invention will be more fully understood from the following detailed description, when read together with the accompanying drawings, in which:

Figure 1 is a longitudinal cross section of an annular graft according to the present invention.

Figure 2 is a cross sectional view of the graft depicted at figure 1.

Figure 3 is a schematic representation of the flow velocity profile in a conventional graft as well as in a graft having a flow deflector.

Figure 4 is a graphic that shows the relative wall shear stress as a function of the quotient of the deflector's radius on the graft's radius.

Figure 5 is cross sectional view of an alternate embodiment of the graft according to the invention having only one stabilizing rod.

Figure 6 is a cross sectional view of another embodiment of the graft having four stabilizing rods.

Figure 7 is a longitudinal cross section of another embodiment of the annular graft in which the flow deflector element is constituted by a plurality of discrete elements.

Figure 8 is a schematic view of a graft according to the invention when joined to a vessel or artery.

Figure 9 shows another embodiment of the graft once sutured to an artery or vessel, where one end of the graft presents a reduced diameter.

The majority of artificial grafts used today in bypass surgery have a cylindrical form with a circular cross section. These grafts, once implanted, develop a layer of disorganized protein and cell remnants, called pseudointima. At the two anastomotic ends, smooth muscle cell proliferation leads to a neointimal formation usually covered by endothelial cells. Excessive thickening of pseudointima and neointima is believed to play a role in limiting graft patency.

Blood flow-induced wall shear stress (internal wall friction) appears to be an important factor determining both pseudointima and neointima thickness. Kraiss et al. (Arteriosclerosis and Thrombosis, 1991) implanted an aorto-aortic together in tandem with a pair of aorto-iliac porous PTFE grafts in baboons, so that the proximal graft supplied flow to the two distal grafts. All grafts had an internal diameter of 5mm. Flow and thus wall shear stress in the proximal graft was twice as much as in the distal grafts. Three months after implantation, neointimal cross-sectional area was half as large in proximal graft as in the distal grafts. Smooth muscle cell proliferation and smooth muscle cell volume were also 50% less in proximal grafts. Kraiss et al concluded that elevated but physiological shear stress inhibits smooth muscle cell proliferation and neointimal thickening in artificial grafts. Further, high shear stress appears to increase graft endothelialization, decrease flow-surface thrombus and induce a thinner surface lining.

The majority of artificial grafts used today have a cylindrical form with a circular cross section. This means that the wall shear stress, $\tau_s$ is given by the Poiseuille's law:

$$\tau_s = \frac{4\mu}{\pi r_s^3}\, Q \tag{1}$$

where $\mu$ is the blood viscosity, Q is the mean blood flow rate and r the internal radius of the graft.

The blood flow rate is determined primarily by the resistance of the vascular bed distal to the graft, Rp. Graft's resistance is given as

$$R_p = \frac{8\,\mu L_s}{\pi r_s^4} \tag{2}$$

which can be significant and flow-limiting when the radius r is small or the graft length, $L_s$, becomes large. Since the graft length is defined by the surgical procedure, the only free parameter for graft geometry is its radius $r_s$.

Grafts with small radius have high resistance (equation 2) and high shear stress (equation 1) and tend to be thrombogenic. Grafts with larger radius, by virtue of equation 1, have a substantially lower wall shear stress and tend to develop a thick pseudointima or neointima layer, which in turn reduces the diameter and lowers patency rates.

From the above analysis, the ideal vascular graft would be a large diameter graft with an increased wall shear stress. Any geometrical adaptation in the form of a graft that leads to an increased wall shear stress while keeping graft cross-sectional dimensions large could be beneficial to graft's patency.

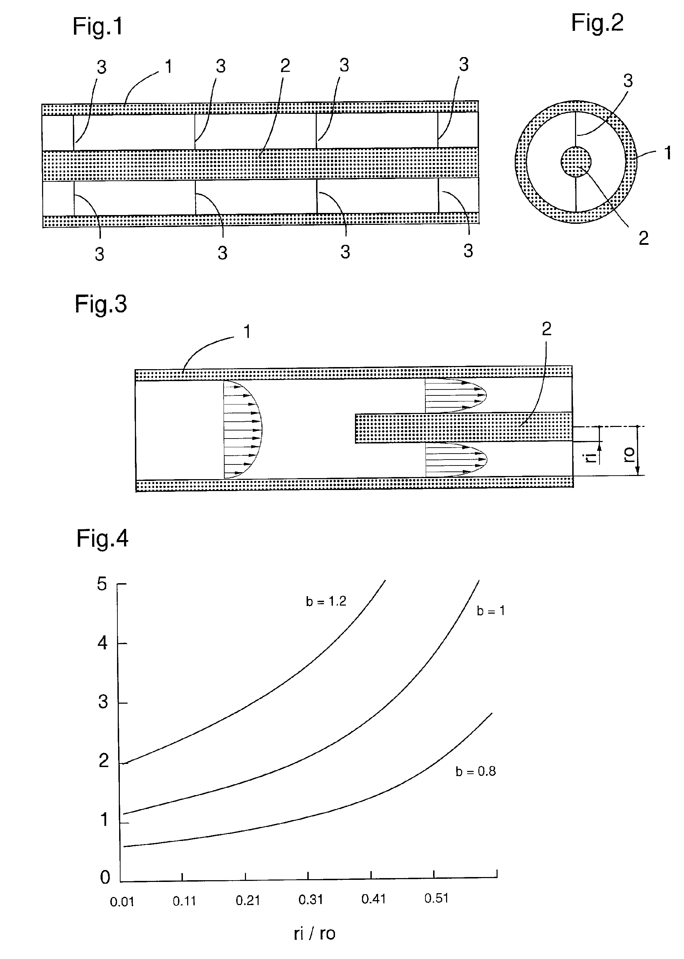

The annular graft configuration object of the invention is proposed here as one of the solutions leading to a graft with increased wall shear stress and is illustrated at figure 1. The annular graft shown is constituted of an annular tubular body 1 of internal radius $r_o$ containing an internal flow deflector element 2 of radius $r_i$. The internal flow deflector 2 shown at figure 2 has a general cylindrical shape but could also have different configurations, it may for example have an elliptical or polygonal cross section or other configuration. The deflector 2 is placed along the longitudinal axis of the graft 1 and secured in this position by means of stabilizing rods 3.

Preferably, the flow deflector 2 is flexible and made out of a material (i.e., PTFE, elastomer or other), which allows to follow easily any flexion of the tubular body 1 while securing the flow deflector element 2 substantially in a central position along the longitudinal axis of the tubular body 1 of the graft. In the embodiment depicted at figures 1 and 2, the flow deflector element 2 extends over the whole length of the graft.

The stabilizing rods 3 are also made in a bio compatible material such as PTFE, DACRON or implantable bio compatible alloys like stainless steel or Nitinol for instance.

Neglecting the influence of the stabilizing rods and assuming fully developed laminar flow with blood taken as a Newtonian fluid of viscosity $\mu$, the velocity profile in the annular graft is given by the following equation:

$$u(r) = \frac{1}{4\mu} \frac{\partial P}{\partial x} \left[ r^2 - r_o^2 + \frac{r_i^2 - r_o^2}{\ln\left(\frac{r_o}{r_i}\right)} \ln\left(\frac{r}{r_i}\right) \right] \qquad (3)$$

An approximate velocity profile is schematically represented at figure 3 in two sections of a graft. A first section, on the left of the figure having no deflector element like in traditional grafts and a second section, on the right of the figure, where a central flow deflector is present. A noticeable increase of the velocity of the blood flow may be noticed in the section having a flow deflector

The wall shear stress on the internal wall as a function of the flow is given by:

$$\tau_{AFG} = -\frac{2\mu}{\pi} \frac{Q}{r_o^4 - r_i^4 - \frac{\left(r_o^2 - r_i^2\right)^2}{\ln\left(\frac{r_o}{r_i}\right)}} \left[ 2r_o + \frac{1}{r_o} \frac{r_i^2 - r_o^2}{\ln\left(\frac{r_o}{r_i}\right)} \right] \qquad (4)$$

Assuming that blood flow through the graft 1 is basically determined by peripheral resistances and not by the graft resistance, the increase in wall shear stress relative in the annular graft of the present invention as compared to the straight conventional graft (i.e without flow deflector) is given as

$$\frac{\tau_{AFG}}{\tau_s} = b^3 \; \frac{1+\dfrac{1-\gamma^2}{2\ln(\frac{1}{\gamma})}}{1-\gamma^4-\dfrac{(1-\gamma^2)^2}{\ln(\frac{1}{\gamma})}} \tag{5}$$

where $\gamma = \dfrac{r_I}{r_o}$ is the ratio of the radius of the flow deflector element on the internal radius of an annular graft according to the invention and $b = \dfrac{r_s}{r}$ is the ratio of the internal radii of a conventional graft.

Figure 4 shows the dependence of the relative wall shear stress increase in the annular graft objet of the invention on parameters $b$ and $\gamma$. The diagram depicted in figure 4 shows the relative wall shear stress ($\tau_{AFG}/\tau_s$) as a function of $\gamma$ for different values of graft radii ratios.

Based on the above analysis, one can notice that for a deflector radius equal to 1/3 of the outside radius, the increase in wall shear stress in the annular graft of the present invention in comparison to a conventional straight graft of equal internal diameter (b = 1) is by a factor 2.2 or 220%, which is substantial. Conversely, one can use an annular graft according to the invention with a larger internal diameter than a conventional graft (b = 0.8) and still keep wall shear stress levels higher than those of a conventional graft using a deflector with a radius equal to 1/3 of the outside radius $r_o$.

The ratio between the radius of the deflector $r_i$ and the internal radius of the tubular body $r_o$ is comprised between 0.1 and 0.8, preferably 0.3.

At figure 1, there are two diametrically opposed stabilizing rods for maintaining the flow deflector 2, however the number of stabilizing rods 3 in a specific cross-section can be variable as well as their repartition.

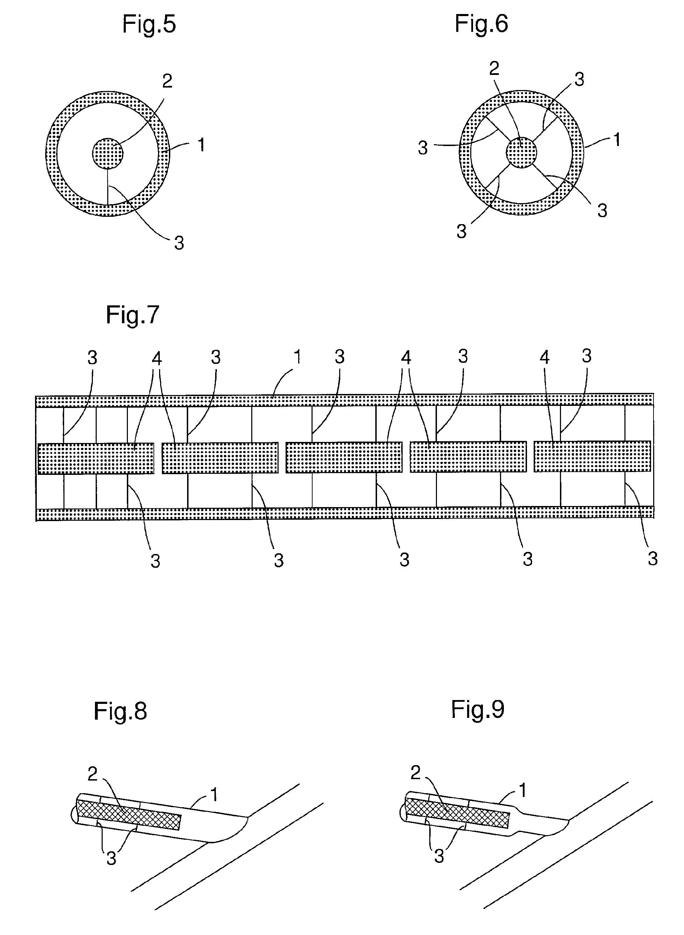

Figure 5 shows an alternate embodiment of the graft according to the invention where only one semi-rigid rod 3 is used to maintain the flow deflector element 2 substantially in the center of the graft's tubular body 1.

Figures 6 depicts another example of an embodiment of the vascular graft in which the flow deflector element 2 is maintained in the center of the tubular body 1 thanks to four stabilizing rods 3, equally spaced apart at 90 degrees.

The orientation of the stabilizing rods 3 may differ along the graft length. The longitudinal spacing between each series of stabilizing rods 3, may also vary and be variable. The flow deflector element 2 may not only be of a general cylindrical shape but also a streamlined body or surface which deflects blood flow towards the graft wall and which leads to an increased wall shear stress.

In an other embodiment, the flow deflector element 2 may not only be continuous but constituted of discrete segments 4 as illustrated at figure 7, each segment 4 being maintained and stabilized substantially in the center of the graft body 1 thanks to one or a plurality of stabilizing rods 3.

While the preferred embodiment shows the deflector element maintained substantially in the center of the graft so as to produce a uniform shear stress increase on the internal walls, it is evident that other embodiments may encompass grafts in which the flow deflector element 2 is not maintained concentrically within the tubular body 1 if a non uniform shear stress increase is desired.

With reference to figure 8, the flow deflector element 2 does not extend over the entire graft length, but stops a small sufficient distance before one or both ends, so that the graft 1 at one or both of its extremities is free of the deflector and therefore easier for the surgeon to suture on the receiving vessel or artery.

In addition, in the region near the anastomosis where there is no deflector present, the graft may be reduced in diameter to better match the dimensions of the artery to which it is sutured as shown at figure 9.

Thanks to presence of the internal flow deflector 2 in the graft's tubular body 1, a noticeable increase of the shear stress on the internal wall of the graft is achieved and therefore the observed phenomena of obstruction of the graft by remnant cells is considerably reduced.

It will be appreciated by persons skilled in the art that the present invention is not limited by what has been particularly shown and described hereinabove. Rather the present invention may include combinations and sub-combinations of the various features disclosed as well as modifications and extensions thereof which fall under the scope of the following claims.

**Claims**

1.    Graft for replacing or bypassing a damaged blood flow path in a human or animal body by vascular surgery, constituted of an elongated tubular body (1), **characterized in that** it comprises at least one internal flow deflector element (2) maintained substantially in the center of the tubular body (1) by means of at least one stabilizing rod (3).

2. Graft according to claim 1, **characterized in that** the ratio between the radius of the flow deflector element (2) and the radius of the tubular elongated body (1) is comprised between 0.1 and 0.8 preferably 0.3.

3. Graft according to one of the preceding claims, **characterized in that** the flow deflector element (2) presents a generally cylindrical shape.

4. Graft according to one of the preceding claims, **characterized in that** the flow deflector element (2) extends only over one portion of the length of the tubular body (1), leaving at least one end of the tubular body(1) free for facilitating its suture to a vessel or artery.

5. Graft according to one of the preceding claims, **characterized in that** one or both ends of the tubular body (1) have a reduced diameter to better match the diameter of the vessel to which it has to be sutured.

6. Graft according to one of the preceding claims, **characterized in that** the flow deflector element is constituted of discrete segments (4), each segment (4) being maintained in the center of the graft body 1 thanks to at least one stabilizing rods. (3)

7. Graft according to one of the preceding claims, **characterized in that** it is made of polytetrafluoroethylene.

8. Use of graft according to one of the preceding claims in vascular surgery for replacing or bypassing a damaged blood pathway.

Fig.1

Fig.2

Fig.3

Fig.4

Fig.5

Fig.6

Fig.7

Fig.8

Fig.9

**European Patent Office**

**PARTIAL EUROPEAN SEARCH REPORT**

which under Rule 45 of the European Patent Convention shall be considered, for the purposes of subsequent proceedings, as the European search report

Application Number

EP 01 10 4106

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 98 58599 A (STERGIOPULOS NIKOLAOS ;ECOLE POLYTECH (CH)) 30 December 1998 (1998-12-30) * page 7, line 1 - page 8, line 14 * * page 8, line 25 - line 31 * * claims 2,3 * | 1-3,6 | A61F2/06 |
| Y | | 4 | |
| Y | FR 2 748 198 A (BRAUN CELSA SA) 7 November 1997 (1997-11-07) * figure 2 * * page 6, line 21 - line 26 * | 4 | |
| Y | FR 2 779 340 A (DELAB) 10 December 1999 (1999-12-10) * figures 2,3 * * page 14, line 26 - page 15, line 28 * * claim 1 * | 1,3,7 | |
| | -/-- | | |

**TECHNICAL FIELDS SEARCHED (Int.Cl.7)**

A61F

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

1-7

Claims searched incompletely :

Claims not searched :

8

Reason for the limitation of the search:

Article 52 (4) EPC - Method for treatment of the human or animal body by surgery

| Place of search | Date of completion of the search | Examiner |
|---|---|---|
| THE HAGUE | 11 June 2001 | Mary, C |

EPO FORM 1503 03.82 (P04C07)

**European Patent Office** · **PARTIAL EUROPEAN SEARCH REPORT** · Application Number

EP 01 10 4106

| Category | Citation of document with indication, where appropriate, of relevant passages | Relevant to claim | CLASSIFICATION OF THE APPLICATION (Int.Cl.7) |
|---|---|---|---|
| Y | WO 00 09059 A (PRODESCO) 24 February 2000 (2000-02-24) * figures 1,6-9 * * page 8, line 19 - page 9, line 5 * * page 11, line 15 - line 31 * | 1,3,7 | |
| A | | 4,5 | |
| A | WO 00 53118 A (MINDGUARD LTD ;YASSOUR YUVAL (IL); YODFAT OFER (IL); GRAD YGAEL (I) 14 September 2000 (2000-09-14) * page 9, line 15 - line 20 * * page 10, line 5 - page 12, line 2 * * page 12, line 9 - page 13, line 2 * | 1 | |
| A | ENDOART, 'Online! XP002169269 Retrieved from the Internet: <URL:http://www.endoart.ch/> 'retrieved on 2001-06-08! * page PRODUCTS, paragraph HELIGRAFT * | 1 | TECHNICAL FIELDS SEARCHED (Int.Cl.7) |
| A | WO 00 74598 A (SCIMED LIFE SYSTEMS INC ;STAUDENMEIER MARIANNE W (US); NUNEZ JOSE) 14 December 2000 (2000-12-14) * figures 4B,5 * * page 6, line 11 - line 23 * * page 7, line 1 - page 8, line 21 * | 1,5 | |

DOCUMENTS CONSIDERED TO BE RELEVANT

EPO FORM 1503 03.82 (P04C10)

**ANNEX TO THE EUROPEAN SEARCH REPORT
ON EUROPEAN PATENT APPLICATION NO.**                     EP 01 10 4106

This annex lists the patent family members relating to the patent documents cited in the above-mentioned European search report.
The members are as contained in the European Patent Office EDP file on
The European Patent Office is in no way liable for these particulars which are merely given for the purpose of information.

11-06-2001

| Patent document cited in search report | | Publication date | Patent family member(s) | | Publication date |
|---|---|---|---|---|---|
| WO 9858599 | A | 30-12-1998 | AU | 730691 B | 15-03-2001 |
| | | | AU | 7669498 A | 04-01-1999 |
| | | | BR | 9810208 A | 08-08-2000 |
| | | | CN | 1261261 T | 26-07-2000 |
| | | | EP | 0989830 A | 05-04-2000 |
| FR 2748198 | A | 07-11-1997 | EP | 0836452 A | 22-04-1998 |
| | | | WO | 9741804 A | 13-11-1997 |
| | | | FR | 2748199 A | 07-11-1997 |
| | | | JP | 11509131 T | 17-08-1999 |
| | | | US | 6059821 A | 09-05-2000 |
| | | | US | 6036723 A | 14-03-2000 |
| FR 2779340 | A | 10-12-1999 | AU | 4045499 A | 20-12-1999 |
| | | | EP | 1082068 A | 14-03-2001 |
| | | | WO | 9962426 A | 09-12-1999 |
| WO 0009059 | A | 24-02-2000 | US | 6159239 A | 12-12-2000 |
| | | | AU | 5670399 A | 06-03-2000 |
| | | | US | 6164339 A | 26-12-2000 |
| | | | US | 6123115 A | 26-09-2000 |
| | | | US | 6192944 B | 27-02-2001 |
| WO 0053118 | A | 14-09-2000 | AU | 3187600 A | 28-09-2000 |
| | | | AU | 3187800 A | 28-09-2000 |
| | | | WO | 0053119 A | 14-09-2000 |
| WO 0074598 | A | 14-12-2000 | AU | 6402600 A | 28-12-2000 |

EPO FORM P0459

For more details about this annex : see Official Journal of the European Patent Office, No. 12/82